## Europäisches Patentamt

## European Patent Office

## Office européen des brevets

(11) Veröffentlichungsnummer: **0 069 852**
**B1**

(12) **EUROPÄISCHE PATENTSCHRIFT**

(45) Veröffentlichungstag der Patentschrift:
**02.05.85**

(21) Anmeldenummer: **82104629.9**

(22) Anmeldetag: **27.05.82**

(51) Int. Cl.⁴: **C 08 G  18/22,** C 08 G  18/66,
B 01 D  13/04, A 61 M  1/16,
A 61 L  2/02

(54) **Linear segmentierte Polyurethane, Verfahren zu deren Herstellung und deren Verwendung.**

(30) Priorität: **11.07.81 DE 3127464**

(43) Veröffentlichungstag der Anmeldung:
**19.01.83 Patentblatt 83/3**

(45) Bekanntmachung des Hinweises auf die Patenterteilung:
**02.05.85 Patentblatt 85/18**

(84) Benannte Vertragsstaaten:
**AT BE CH FR GB IT LI LU NL SE**

(56) Entgegenhaltungen:
**DE - A - 2 461 363**
**DE - B - 1 222 248**
**GB - A - 2 049 544**

(73) Patentinhaber: **Akzo GmbH,**
**Postfach 10 01 49 Kasinostrasse 19-23,**
**D-5600 Wuppertal-1 (DE)**

(72) Erfinder: **Behnke, Joachim, Dr. Dipl.-Ing., Zu den**
**Weizenäckern 26, D-8762 Amorbach (DE)**
Erfinder: **Josefiak, Christoph, Dr. Dipl.-Chem.,**
**Frankenstrasse 12e, D-8751 Kleinwallstadt (DE)**

## Beschreibung

Die Erfindung betrifft ein Verfahren zur Herstellung von linear segmentierten Polyurethanen durch gleichzeitiges Umsetzen von Makrodiolen, aromatischen Diisocyanaten und niedermolekularen monomeren Diolen als Kettenverlängerer in einem inerten Lösungsmittel nach dem sogenannten Eintopfverfahren in Gegenwart von Katalysatoren sowie die Verwendung derartiger Polyurethane, insbesondere zur Herstellung von Membranen.

Polyurethane gehören zu einer Polymerklasse, die sich durch eine sehr grosse Vielfalt auszeichnet. So gibt es praktisch unbegrenzte Variationsmöglichkeiten bei deren Herstellung durch entsprechende Auswahl der Ausgangsstoffe. Zahlreiche aliphatische, cycloaliphatische und aromatische Polyisocyanate können allein oder im Gemisch zum Aufbau von Polyurethanen eingesetzt werden. Neben niedermolekularen monomeren Glykolen wie Äthylenglykol, Butylenglykol können auch höhermolekulare Hydroxyendgruppen aufweisende Substanzen wie Polyester, Polyglykole und dergleichen eingesetzt bzw. mitverwendet werden. Durch Verändern des Verhältnisses des niedermolekularen Glykols zum höhermolekularen Glykol lassen sich Polyurethane mit den verschiedensten Eigenschaften gewinnen. Polyurethane, bei denen in der Polymerkette Bausteine vorliegen, die aus dem höhermolekularen Polyester oder dem höhermolekularen Glykol bestehen und die weiterhin mit Segmenten verknüpft sind, welche durch die Reaktion des Diisocyanats mit dem niedermolekularen Glykol wie beispielsweise Äthylenglykol entstanden sind, werden auch Blockpolymere oder segmentierte Polyurethane genannt.

Durch Verwendung von Verbindungen mit mehr als zwei Funktionen, wie z.B. dreiwertigen Alkoholen, kann die Vielzahl der Polyurethane noch vermehrt werden.

Darüber hinaus finden häufig in mehr oder weniger grossem Umfang bei der Synthese der Polyurethane Nebenreaktionen statt, wie z.B. die Bildung von Allophanaten, wodurch gewollt oder ungewollt die Eigenschaften der Polyurethane verändert werden. Die Vielzahl der Möglichkeiten in der Polyurethanchemie ist somit praktisch unüberschaubar.

Von Einfluss bei der Herstellung der Polyurethane ist ferner, ob man die Polyurethane in Masse herstellt, das heisst ohne Mitverwendung eines Lösungsmittels, oder ob man die Umsetzung in einem Lösungsmittel vornimmt.

Beim Arbeiten in einem Lösungsmittel sind wiederum verschiedene Wege möglich, so kann man zunächst durch Umsetzung eines Makrodiols mit dem Diisocyanat ein sogenanntes Präpolymer herstellen und dies sodann mit dem Kettenverlängerer umsetzen. Daneben ist das sogenannte Eintopfverfahren zu erwähnen, bei dem alle Ausgangsstoffe, d.h. das Polyisocyanat, das Makrodiol und das niedermolekulare Glykol gleichzeitig umgesetzt werden. Weitere Möglichkeiten der Variation bieten sich dadurch an, dass man beim Eintopfverfahren das Verhältnis von NCO zu OH-Gruppen während der Hauptreaktion unterschiedlich wählt.

Bei nichtkatalysierten Eintopfreaktionen muss man häufig mit einem Überschuss an Diisocyanat arbeiten um zu den gewünschten Molgewichten zu kommen; hierdurch werden aber die gefürchteten Nebenreaktionen gefördert, z.B. die Allophanat- und Biuretbildung. Dadurch wird die Reproduzierbarkeit der Ansätze beeinträchtigt, auch kann es zu Entmischungen kommen.

Zu den zahllosen oben erwähnten Variationsmöglichkeiten, die man durch die Wahl der Ausgangsstoffe usw. hat, kommen weitere Beeinflussungsfaktoren, die man durch den Einsatz der verschiedensten Katalysatoren hat. Es sind zahlreiche Katalysatoren bekannt, die bei der Herstellung von Polyurethanen eingesetzt worden sind. Man kann mit ihnen nicht nur die Geschwindigkeit der eigentlichen Polyurethanbildung steuern, sondern auch gewollt oder ungewollt die Eigenschaften des entstehenden Produkts verändern. So wird häufig durch die Verwendung unterschiedlicher Katalysatoren das Molekulargewicht bei der Herstellung der Polyurethane verändert. Auch wird die Molekulargewichtsverteilung beeinflusst. Nicht zuletzt katalysieren die verwendeten Katalysatoren nicht nur die eigentliche Polyurethanbildung, sondern häufig auch mehr oder weniger die verschiedensten Nebenraktionen wie Allophanatbildung, Trimerisierung und dergleichen, die zu verzweigten, vernetzten und damit im allgemeinen schwer oder sogar unlöslichen Produkten führen.

Für Massenprodukte kann man häufig innerhalb verhältnismässig weiter Grenzen ein Schwanken der Eigenschaften des eingesetzten Polyurethans in Kauf nehmen. Für viele Einsatzzwecke hingegen ist ein Produkt mit möglichst gleichbleibenden und genau einstellbaren Eigenschaften erwünscht. Dies trifft insbesondere für das Gebiet der Membranen zu, deren Herstellung aus einem fertigen Polymer bereits eine verhältnismässig komplizierte Technologie fordert. So werden die Eigenschaften von Membranen durch Abweichung in der Dicke der Folien, durch die Koagulationsbedingungen, durch ihre Struktur bzw. in grossem Masse beeinflusst, so dass man nicht noch einen weiteren Unsicherheitsfaktor, nämlich den Aufbau des Polymerisats in Kauf nehmen kann. Man ist vielmehr an Polymeren mit konstanter chemischer Struktur und konstanten Eigenschaften interessiert.

Man hat bereits versucht, Lösungen von linear segmentierten Polyurethanen mit konstanter Viskosität herzustellen, indem man dem Reaktionsgemisch entweder von vornherein oder bei einer bestimmten Stufe der Umsetzung sogenannte Kettenabbruchmittel und Stabilisatoren zusetzt. Auf diese Weise ist es jedoch nicht möglich, reproduzierbar Polyurethane mit gleicher und möglichst enger Molekulargewichtsverteilung zu erhalten. Auch durch Variationen der Reaktionstemperatur kann dieses Ziel nur in unbefriedigendem Masse erreicht werden. So dauern die Umsetzungen bei niedrigen Temperaturen viel zu lange, um eine gewerblich verwertbare Herstellung von Polyurethanlösungen zu erlauben. Bei höheren Temperaturen treten Nebenreaktionen und Abbaureaktionen in unerwünschtem Masse auf. Auch durch den Einsatz von einer ganzen Reihe von Katalysatoren ist es bisher nicht gelungen, dieses Problem zu lösen. Zahlreiche Katalysatoren, insbesondere die sehr häufig verwendeten tertiären Aminverbindungen, weisen in polaren Lösungsmitteln bei höheren Temperaturen keine katalytische Wirksamkeit mehr auf. Viele Katalysatoren, wie z.B. organische Zinnverbindungen, sind giftig und daher für den medizinischen Einsatzbereich von Polyurethanmembranen wenig geeignet.

In dem Aufsatz von L. Thiele «Isocyanatreaktion und Katalyse in der Polyurethanchemie» Act. Polymerica 30 (1979) Heft 6, S. 232 bis 242, werden zahlreiche Katalysatoren erwähnt, die bei der Herstellung von Polyurethanen Verwendung finden können. Mit den dort genannten Katalysatoren werden nicht die Vorteile der Erfindung erreicht.

In der US-A-3 769 245 wird die Herstellung von Polyurethanschäumen in der Schmelze beschrieben; dabei sollen Isocyanatgruppen mit Carboxylgruppen reagieren, wozu als Katalysator vorzugsweise Magnesiumacetat verwendet werden soll. Magnesiumacetat ist jedoch als Katalysator für das erfindungsgemässe Verfahren völlig ungeeignet.

Als Säuren, welche mit den verschiedensten Metallen Salze bilden, die als Katalysator für das dort beschriebene Verfahren geeignet sein sollen, werden vorwiegend aliphatische Säuren genannt, die ebenfalls keine für das erfindungsgemässe Verfahren brauchbare Katalysatoren ergeben. Ein Hinweis, dass die am Rande erwähnten Benzoate und Naphthate, insbesondere Calcium- oder Magnesiumbenzoat oder -naphthat die Herstellung von Polyurethanen bzw. Polyurethanlösungen mit den gewünschten Eigenschaften ermöglichen sollen, ist dieser Patentschrift jedoch nicht zu entnehmen.

Obwohl bereits zahlreiche Verfahren zur Herstellung von Polyurethanen bekannt sind, besteht noch ein Bedürfnis nach verbesserten Verfahren zur Herstellung von Polyurethanen, die sich insbesondere durch eine enge Molekulargewichtsverteilung auszeichnen.

Aufgabe der Erfindung ist deshalb ein Verfahren zur Herstellung von linear segmentierten Polyurethanen, nach dem sich in reproduzierbarer Weise Polyurethane herstellen lassen, die sich durch eine besonders enge Molekulargewichtsverteilung und hohes Molekulargewicht auszeichnen.

Aufgabe der Erfindung ist es ferner zu ermöglichen, gezielt und reproduzierbar Polyurethane mit einem bestimmten Molekulargewicht und einer entsprechenden Molekulargewichtsverteilung herzustellen. Aufgabe der Erfindung ist es ferner, Polyurethane zur Verfügung zu stellen, die sich besonders vorteilhaft bei der Herstellung von Membranen verwerten lassen, die insbesondere auf dem medizinischen Sektor eingesetzt werden sollen.

Diese Aufgabe wird durch ein Verfahren zur Herstellung von linear segmentierten Polyurethanen durch gleichzeitige Umsetzung von Makrodiolen, niedermolekularen Diolen als Kettenverlängerer und aromatischen Diisocyanaten in einem Lösungemittel in Gegenwart von Katalysatoren gelöst, das dadurch gekennzeichnet ist, dass man in Gegenwart von katalytischen Mengen eines Magnesium- oder Calciumsalzes einer aromatischen Carbonsäure, Makrodiole, monomere, niedermolekulare Diole und aromatische Diisocyanate bei einer Temperatur von ca. 70 bis 120°C in einem inerten Lösungsmittel umsetzt, wobei die Konzentration der Ausgangsstoffe etwa 10 bis 40 Gew.-% beträgt, bezogen auf das Gemisch Ausgangsstoffe und Lösungsmittel und man das Makrodiol in solchen Mengen verwendet, dass sich im fertigen Polyurethan ein Gewichtsverhältnis von Weichsegment zu Hartsegment von etwa 4 : 1 bis 1 : 4 einstellt. Als niedermolekulare Diole sind besonders Alkylenglykole mit 2 bis 4 Kohlenstoffatomen geeignet.

Vorzugsweise verwendet man die Ausgangsstoffe in einer Konzentration von etwa 15 - 30 Gew.-%. Als aromatisches Diisocyanat ist 4,4'-Diphenylmethandiisocyanat besonders geeignet. Günstige Katalysatoren sind Salze von aromatischen Monocarbonsäuren, von denen die Benzoesäure, die Pyridin-2-carbonsäure und die Thiophen-2-carbonsäure besonders hervorzuheben sind.

Ein sehr geeignetes Lösungsmittel für die Umsetzung sind e-Butyrolacton und Dimethylacetamid.

In einer besonders vorteilhaften Ausführungsform des erfindungsgemässen Verfahrens werden als Makrodiole Polyalkylenglykole, vorzugsweise Polyäthylenglykol und Polytetramethylenglykol verwendet. Ein zweckmässiger Kettenverlängerer ist Äthylenglykol.

Die Polyurethanlösungen weisen vorteilhafterweise eine molekulare Uneinheitlichkeit von $U = \overline{M}_w : \overline{M}_n$ von etwa 2 bis 7, vorzugsweise von etwa 2 bis 5 auf. Ihr mittleres Molekulargewicht $\overline{M}_w$ liegt vorzugsweise im Bereich von etwa 60.000 - 250.000 insbesondere 100.000 bis 150.000.

Die erfindungsgemässen Polyurethane sind vor allem zur Herstellung von Membranen, insbesondere von Membranen für die Sterilfiltration und Blutoxygenation geeignet.

Die Durchführung des Verfahrens zur Herstellung der Polyurethane kann nach an sich bekannten Methoden erfolgen. Sie wird nach dem sogenannten Eintopfverfahren durchgeführt, für das u.a. eine besonders vorteilhafte Ausführungsform in der DE-A-2 409 789 beschrieben wird, auf die sich hier ausdrücklich bezogen wird.

Es versteht sich von selbst, dass zur Herstellung der Polyurethanlösungen möglichst reine Ausgangssubstanzen und Lösungsmittel eingesetzt werden. So sollte das verwendete Diisocyanat einen Gehalt von mindestens 99,0, vorzugsweise mindestens 99,5% aufweisen. Das Diisocyanat sollte nicht dimerisiert und/oder trimerisiert sein.

Besonders geeignet als verwendbares aromatisches Diisocyanat ist Diphenylmethan-4,4'-diisocynat. Eingesetzt werden können u.a. auch 1,4-Phenylendiisocyanat und Toluylendiisocyanat.

Die aromatischen Diisocyanate können allein oder auch im Gemisch verwendet werden. In bestimmten Fällen ist es auch möglich, einen Teil des aromatischen Diisocyanats durch aliphatische oder cycloaliphatische Diisocyanate zu substituieren.

Als Makrodiole können übliche Polymere mit zwei vorzugsweise endständigen Hydroxylgruppen verwendet werden. Dazu sind insbesondere Polyester und Polyäther zu zählen. Vorzugsweise werden Polyalkylenglykole, insbesondere Polyäthylenglykol und Polytetramethylenglykol verwendet. Das eingesetzte Makrodiol weist zweckmässigerweise ein Molekulargewicht von etwa 600 bis 20 000 auf. Vorzugsweise beträgt das Molekulargewicht des Makrodiols etwa 1 000 bis 6 000.

Der Katalysator wird im allgemeinen in üblichen katalytischen Mengen eingesetzt. Besonders überraschend war es, dass es vielfach ausreicht, im Rahmen der Erfindung mit lediglich einigen ppm zu arbeiten. Die Mengenangaben bezüglich des Katalysators werden im Rahmen der Erfindung auf das Gesamtausgangsgemisch bezogen, d.h. auf die eingesetzten Ausgangsstoffe einschliesslich des verwendeten Lösungsmittels. Die Gewichtsangabe bei dem Katalysator bezieht sich auf Menge Calcium- bzw. Magnesiummetall. In vielen Fällen kann es ausreichend sein, wenn man mit etwa 15 ppm Katalysator arbeitet.

Durch Variieren der Menge des eingesetzten Katalysators kann man neben der Geschwindigkeit der Umsetzung auch die Eigenschaften des entstehenden Polyurethans steuern. Im allgemeinen wird durch Erhöhung des Anteils des zugesetzten Katalysators die Einheitlichkeit des entstehenden Polymers verbessert. Die gewünschten engen Polymerverteilungen erhält man, wenn die Menge des Katalysators vorzugsweise in einem Bereich von etwa 10 bis 200 ppm liegt.

Die Menge des eingesetzten Katalysators hängt auch vom verwendeten Lösungsmittel ab. So ist im allgemeinen bei amidischen Lösungsmitteln wie z.B. Dimethylformamid, Dimethylacetamid, N-Methylpyrrolidon usw. die benötigte Menge höher als bei nicht-amidischen Lösungsmitteln wie z.B. γ-Butyrolacton. Für amidische Lösungsmittel werden vorzugsweise 75 - 200 ppm, für nicht amidische Lösungsmittel vorzugsweise ca. 15 - 50 ppm Katalysator verwendet. Selbstverständlich können auch noch höhere Mengen eingesetzt werden; meistens ist es jedoch wenig sinnvoll, mehr als 1 000 ppm Katalysator zu verwenden.

Als aromatische Carbonsäuren, die mit Magnesium bzw. Calcium als Katalysator gemäss der vorliegenden Erfindung verwendbare Salze bilden, sind vor allem zu nennen Benzoesäure, 4-Nitrobenzoesäure, 4-Dimethylaminobenzoesäure, Pyridin-2-carbonsäure, Pyridin-3-carebonsäure, 4-Pyridin-4-carbonsäure, ferner Salze der verschiedenen Thiophensäuren wie Thiophen-2-carbonsäure, Thiophen-3-carbonsäure, Furancarbonsäure, Pyrrolcarbonsäuren, ferner Naphthalincarbonsäuren u. dgl.

Die als Katalysator eingesetzten Salze können nach üblichen Verfahren gewonnen werden, so z.B., indem man von dem Metalloxid, z.B. Magnesiumoxid und der Säure ausgeht und die Salzbildung gegebenenfalls in Gegenwart von Wasser oder bei erhöhter Temperatur durchführt. Anschliessendes Umkristallisieren ist häufig zweckmässig. Da, wie bereits erwähnt, die chemischen Reaktionen, die bei der Herstellung von Polyurethanen ablaufen, durch Katalysatoren sehr stark beeinflusst werden können, versteht es sich von selbst, dass im Rahmen der Erfindung die eingesetzten Katalysatoren möglichst rein und insbesondere frei von freien Säuren sind. Zweckmässig ist der Reinheitsgrad des Katalysators höher als 95%.

Die verwendeten Lösungsmittel sind inert, d.h. sie sollen im wesentlichen weder mit den Ausgangsstoffen, d.h. den Diisocyanat- und den Dihydroxyverbindungen noch mit dem sich bildenden Polyurethan und dem verwendeten Katalysator reagieren können. Geeignete Lösungsmittel sind u.a. Dimethylacetamid, γ-Butyrolacton und Dimethylformamid.

Die Temperatur, bei der die Umsetzung durchgeführt wird, liegt zwischen etwa 70 bis 120°C. Bei niedrigeren Temperaturen besteht die Gefahr, dass das Polyurethan vor Abschluss der Reaktion ausfällt, bei Temperaturen über 120°C kann es zu unerwünschten Nebenreaktionen und zu einem Abbau des Polyurethans kommen. Vorzugsweise wird die erfindungsgemässe Reaktion in einem Temperaturbereich von etwa 70 - 90°C durchgeführt. Die zu wählende Temperatur hängt auch von dem verwendeten Lösungsmittel ab. So liegt die zu wählende Temperatur bei einem Einsatz von Dimethylacetamid meistens etwas niedriger als bei einem Einsatz von γ-Butyrolacton. So ist für γ-Butyrolacton ein bevorzugter Bereich 80 - 90°, für Dimethylacetamid 60 bis 80°C.

Als Makrodiole können z.B. Polyester wie Polyäthylenadipat eingesetzt werden. Besonders geeignet sind Polyalkylenglykole wie Polyäthylen- und Polytetramethylenglykol.

Als Kettenverlängerer werden vorzugsweise die monomeren, niedermolekularen Diole Äthylenglykol und Butylenglykol-(1.4) eingesetzt. Auch andere Diole wie Hexamethylenglykol sind geeignet.

Unter einem monomeren, niedermolekularen Diol sind im Gegensatz zu den Makrodiolen nichtpolymere Diole wie z.B. die aliphatischen Verbindungen Äthylenglykol, Hexamethylenglykol, Butandiol-(1.4) oder -(1.3) zu verstehen.

Das Verhältnis von Weichsegment zu Hartsegment wird durch die Wahl des Verhältnisses von Makrodiol : Kettenverlängerer eingestellt. So können durch den Anteil von Weichsegment die mechanischen Eigenschaften der hergestellten Membranen wie Festigkeit, Elastizität u. dgl. beeinflusst werden. Auch hängen Transporteigenschaften wie die Permeabilität davon ab.

Es empfiehlt sich, zu Ende der Umsetzung bzw. kurz vor Abschluss der Reaktion ein Kettenabbruchmittel und gegebenenfalls Stabilisatoren zuzugeben.

Die bei der Umsetzung erhaltenen Polyurethanlösungen können direkt zur Herstellung der verschiedensten Artikel insbesondere für die Gewinnung von Membranen verwendet werden. Es ist auch möglich, das Polyurethan durch Entfernen des Lösungsmittels oder durch Ausfällen zu isolieren und zu einem späteren Zeitpunkt, z.B. durch Wiederauflösen in einem Lösungsmittel und Formen zu verarbeiten.

Die erhaltenen Polyurethanlösungen werden jedoch vorzugsweise direkt zur Herstellung von Membranen eingesetzt, die auf industriellen Gebieten, z.B. bei Ultrafiltration, Sterilfiltration oder auf medizinischem Gebiet wie z.B. der Blutoxygenation verwendet werden sollen. Ein vorteilhaftes Herstellungsverfahren für derartige Membranen wird in der DE-A-2 918 027 (= GB-A-2 049 544) beschrieben. Aus den gemäss der Erfindung hergestellten Polyurethanlösungen werden Membranen vor allem nach dem Phaseninversionsverfahren hergestellt. Es können sowohl symmetrische als auch asymmetrische Membranen hergestellt werden.

Wird das Polyurethan beispielsweise durch Abdampfen des Lösungsmittels abgetrennt, so kann es bei der Weiterverarbeitung zweckmässig sein, wenn man mit einem Lösungsmittel wie Alkohol gegebenenfalls vorhandene Oligomerenanteile abtrennt. Dies ist bei der Direktverarbeitung der Lösung zur Herstellung von Membranen nach dem Koagulationsverfahren nicht erforderlich, da dabei der Oligomerenanteil nicht in der Membran verbleibt.

Es war besonders überraschend, dass man gemäss der Erfindung Polyurethane auf einfache Weise herstellen kann, die eine sehr enge Molekulargewichtsverteilung besitzen. Besonders überraschend war es, dass man mit Hilfe des erfindungsgemässen Verfahrens reproduzierbar konstante Molekulargewichte einstellen kann. Die Uneinheitlichkeit U, d.h. der Quotient aus dem Gewichtsmittel und dem Zahlenmittel des Molekulargewichts wird gegenüber bekannten Polyurethanen verringert. So ist es ohne weiteres möglich, durch die erfindungsgemässe Verwendung des Katalysators die Uneinheitlichkeit von 15 auf 5 zu reduzieren gegenüber einem Ansatz, der unter sonst gleichen Bedingungen jedoch ohne Katalysator gefahren wird.

Besonders überraschend war es, dass durch den erfindungsgemässen Einsatz der speziellen Katalysatoren Nebenreaktionen weitgehend ausgeschaltet werden und insbesondere die unerwünschte Allophanatbildung, die sonst vor allem gegen Ende der Reaktion auftritt und zu vernetzten Produkten führt, praktisch nicht stattfindet. Dies ist vor allem von Vorteil, wenn man aus den Polyurethanen Membranen herstellen möchte. Die Verwendung von Diisocyanat im Überschuss ist nicht erforderlich.

Sehr vorteilhaft ist ferner, dass man bei der Herstellung von Membranen nach dem Koagulationsverfahren den verwendeten Katalysator völlig oder zumindest zu einem überwiegenden Teil auswaschen kann, so dass man zu praktisch schwermetallfreien Membranen gelangt. Dies ist insbesondere für die medizinische Anwendung der Membranen von höchster Bedeutung.

Aber auch bei Trennprozessen auf industriellem Gebiet z.B. bei der Sterilfiltration, wie sie in der Lebensmittelindustrie vorkommt, spielt dies eine Rolle.

Die Erfindung wird durch folgende Beispiele näher erläutert:

*Beispiel 1*

In einem thermostatisierbaren 3-l-Reaktionsgefäss mit Rührer und Thermometer wurden zunächst 91,44 g Polyäthylenglykol 1000 (0,09 Mole) bei 80 =C im Vakuum während 1 Std. entwässert. Anschliessend wurde 1318 g amin- und wasserfreies Dimethylacetamid, 37,92 g (0,61 Mole) Äthylenglykol und 1,5 g Magnesiumbenzoat (entsprechend 84 ppm Mg, bezogen auf die gesamte Reaktionsmischung) zugegeben und die Lösung auf 75°C aufgeheizt. Zu diesem Gemisch wurden innerhalb von 5 Minuten 175,4 g (0,7 Mole) 4,4'-Diphenylmethandiisocyanat in Festsubstanz zugegeben. Die Temperatur in der Lösung stieg kurzfristig auf 90°C, um innerhalb etwa 10 Minuten wieder auf die Thermostattemperatur von 75°C zurückzugehen. Der Anstieg der Viskosität der Reaktionsmischung wurde über die Leistungsaufnahme des Rührmotors verfolgt und mittels eines Schreibens registriert. Im Verlauf weiterer 30 Minuten wurden tropfenweise 5,2 g Diphenylmethandiisocyanat, gelöst in 25 g Dimethylacetamid, zugetropft, bis die Lösung hochviskos war. Die gesamte Reaktionsdauer betrug 40 Minuten. Nach Zugabe von 32 g Äthanol als Kettenabbrecher wurde das entstandene Polyurethan in Wasser gefällt, gea aschen und getrocknet. Durch Streulichtmessung in Dimethylformamid wurde das Molgewicht zu 144 000 Dalton gefunden, die relative Viskosität in Dimethylformamid betrug 1,68 und die Uneinheitlichkeit, gemessen mit einem Waters-Gelpermeationschromatographen Modell 200 unter Verwendung von 4 Styragelsäulen, betrug

$$U = \frac{\overline{M}_w}{\overline{M}_n} = 4,1.$$

Die relativen Viskositäten werden in Dimethylformamid bei 25°C bestimmt, wobei 0,5 g Substanz aufgelöst und auf 100 ml aufgefüllt werden.

*Vergleichsbeispiel*

Dieses Beispiel zeigt den Unterschied zwischen dem erfindungsgemässen Verfahren und einem Verfahren, bei dem ein anderer Katalysator eingesetzt wird.

*Beispiel 2*

Es wurde ein Polyurethanansatz analog Beispiel 1 hergestellt, jedoch wurden anstelle von Mg-benzoat als Katalysator 0,73 g Li-benzoat (entsprechend 20 ppm Li, bezogen auf die gesamte Reaktionsmischung) zugesetzt. Die Reaktionsdauer betrug insgesamt 1 Stunde. Dabei wurde praktisch kein Viskositätsanstieg beaobachtet. Das aus der Reaktionsmischung isolierte Polyurethan zeigte im Gelpermeationschromatogramm ein Maximum der Molgewichtsverteilung bei etwa 40 000 Dalton und eine Uneinheitlichkeit von 6,9, während die relative Viskosität, in Dimethylformamid gemessen, 1,30 betrug.

Weitere Beispiele sind der folgenden Tabelle zu entnehmen.

| Bei-spiele Nr. | Katalysator | Kata-lysator-menge (g) | Makro-diol Mol-gew. | Ansatzmengen Menge (g) | Äthy-len-glykol (g) | Lösungs-mittel (g) | Dime-thyl-methan-diiso-cyanat (g) | Temp. °C | Vis-kosi-tät n rel | $\overline{M}_w$ | $U = \frac{\overline{M}_w}{\overline{M}_n}$ |
|---|---|---|---|---|---|---|---|---|---|---|---|
| 3 | $(O_2N\text{-}\langle \rangle\text{-}COO)_2Mg$ | 0,5065 | Poly-äthylen-glykol MG 1000 | 129,9 | 53,9 | Dimethyl-acetamid 1791,95 | 266,7 (107%) | 75 | 1,60 | 172 000 | 7,8 |
| 4 | $(O_2N\text{-}\langle \rangle\text{-}COO)_2Mg$ | 2,21 | » | 129,9 | 53,9 | 1791,2 | 269,2 | 75 | 1,59 | 121 000 | 4,3 |
| 5 | $(\langle\langle\rangle\rangle\text{-}COO)_2Mg$ | 2,27 | » | 124,9 | 53,9 | 1741,0 | 264,8 (106,25) | 77 | 1,64 | 158 000 | 5,8 |
| 6 | $(\langle \rangle\text{-}COO)_2Mg$ (S) | 1,71 | » | 129,9 | 53,9 | 1866,4 | 266,7 | 76 | 1,75 | 160 000 | 5,0 |
| 7 | $(\langle \rangle\text{-}COO)_2Mg$ (O) | 1,53 | » | 129,9 | 53,9 | 1791,9 | 264,2 | 74 | 1,85 | 207 000 | 6,2 |
| 8 | $(\langle \rangle\text{-}COO)_2Mg$ (N) | 1,66 | » | 129,9 | 53,9 | 1791,9 | 274,1 | 74 | 1,68 | 183 000 | 6,2 |
| 9 | $(\langle \rangle\text{-}COO)_2Mg$ | 1,65 | Polytetra-methylen-glykol MG 1000 | 129,9 | 53,9 | 1791,2 | 279,2 | 75 | 1,61 | 110 000 | 3,8 |
| 10 | $(\langle \rangle\text{-}COO)_2Mg$ | 0,308 | Polyäthy-lenglykol MG 1000 | 152,4 | 37,86 | $\gamma$-Butyro-lacton 1647,0 | 194,6 | 90 | 1,49 | 63 000 | 3,7 |
| 11 | $(\langle \rangle\text{-}COO)_2Mg$ | 0,308 | » | 152,4 | 37,86 | 1647,0 | 198,4 | 90 | 1,58 | 126 000 | 4,8 |

**Patentansprüche für die Vertragsstaaten:**
BE, CH, FR, GB, IT, LI, LU, NL, SE

1. Verfahren zur Herstellung von linear segmentierten Polyurethanen durch gleichzeitige Umsetzung von Makrodiolen, niedermolekularen Diolen als Kettenverlängerer und aromatischen Diisocyanaten in einem Lösungsmittel in Gegenwart von Katalysatoren, dadurch gekennzeichnet, dass man in Gegenwart von katalytischen Mengen eines Magnesium- oder Calciumsalzes einer aromatischen Carbonsäure, Makrodiole, monomere, niedermolekulare Diole und aromatische Diisocyanate bei einer Temperatur von ca. 70 bis 120 °C in einem inerten Lösungsmittel umsetzt, wobei die Konzentration der Ausgangsstoffe etwa 10 bis 40 Gew.-% beträgt, bezogen auf das Gemisch Ausgangsstoffe und Lösungsmittel, und man das Makrodiol in solchen Mengen verwendet, dass sich im fertigen Polyurethan ein Gewichtsverhältnis von Weichsegment zu Hartsegment von etwa 4 : 1 bis 1 : 4 einstellt.

2. Verfahren nach Anspruch 1, dadurch gekennzeichnet, dass man als monomere, niedermolekulare Diole Alkylendiole mit 2 bis 4 Kohlenstoffatomen verwendet.

3. Verfahren nach Anspruch 1 bis 2, dadurch gekennzeichnet, dass man die Ausgangsstoffe in einer Konzentration von etwa 15 - 30 Gew.-% verwendet.

4. Verfahren nach den Ansprüchen 1 bis 3, dadurch gekennzeichnet, dass man als aromatisches Diisocyanat 4,4'-Diphenylmethandiisocyanat verwendet.

5. Verfahren nach den Ansprüchen 1 bis 4, dadurch gekennzeichnet, dass man als Katalysator Salze einer aromatischen Monocarbonsäure verwendet.

6. Verfahren nach Anspruch 5, dadurch gekennzeichnet, dass man als Katalysator Salze der Benzoesäure verwendet.

7. Verfahren nach Anspruch 5, dadurch gekennzeichnet, dass man als Katalysator Salze der Pyridin-2-carbonsäure verwendet.

8. Verfahren nach Anspruch 5, dadurch gekennzeichnet, dass man als Katalysator Salze der Thiophen-2-carbonsäure verwendet.

9. Verfahren nach den Ansprüchen 1 bis 8, dadurch gekennzeichnet, dass man als inertes Lösungsmittel $\gamma$-Butyrolacton verwendet.

10. Verfahren nach den Ansprüchen 1 bis 8, dadurch gekennzeichnet, dass man als inertes Lösungsmittel Dimethylacetamid verwendet.

11. Verfahren nach den Ansprüchen 1 bis 10, dadurch gekennzeichnet, dass man als Markodiol Polyalkylenglykole verwendet.

12. Verfahren nach Anspruch 11, dadurch gekennzeichnet, dass man als Polyalkylenglykol Polyäthylenglykol verwendet.

13. Verfahren nach Anspruch 11, dadurch gekennzeichnet, dass man als Polyalkylenglykol Polytetramethylenglykol verwendet.

14. Verfahren nach den Ansprüchen 2 bis 13, dadurch gekennzeichnet, dass man als niedermolekulares, monomeres Diol Äthylenglykol verwendet.

15. Polyurethane erhalten nach einem der Verfahren gemäss den Ansprüchen 1 bis 14, gekennzeichnet durch eine molekulare Uneinheitlichkeit der Polyurethane von $U = \overline{M}_w : \overline{M}_n = 2 - 7$.

16. Polyurethane nach Anspruch 15, gekennzeichnet durch eine molare Uneinheitlichkeit von $U = 2 - 5$.

17. Polyurethane nach den Ansprüchen 15 oder 16, gekennzeichnet durch ein mittleres Molekulargewicht $\overline{M}_w = 60.000$ bis $250.000$.

18. Polyurethane nach Anspruch 17, gekennzeichnet durch ein mittleres Molekulargewicht $\overline{M}_w = 100.000$ bis $150.000$.

19. Verwendung der Polyurethane nach den Ansprüchen 15 bis 18 zur Herstellung von Membranen.

20. Verwendung nach Anspruch 19 zur Herstellung von Membranen für die Sterilfiltration.

21. Verwendung nach Anspruch 19 für die Herstellung von Membranen für die Blutoxigenation.


**Patentansprüche für den Vertragsstaat: AT**

1. Verfahren zur Herstellung von linear segmentierten Polyurethanen durch gleichzeitige Umsetzung von Makrodiolen, niedermolekularen Diolen als Kettenverlängerer und aromatischen Diisocyanaten in einem Lösungsmittel in Gegenwart von Katalysatoren, dadurch gekennzeichnet, dass man in Gegenwart von katalytischen Mengen eines Magnesiumoder Calciumsalzes einer aromatischen Carbonsäure, Makrodiole, monomere, niedermolekulare Diole und aromatische Diisocyanate bei einer Temperatur von ca. 70 bis 120°C in einem inerten Lösungsmittel umsetzt, wobei die Konzentration der Ausgangsstoffe etwa 10 bis 40 Gew.-% beträgt, bezogen auf das Gemisch Ausgangsstoffe und Lösungsmittel, und man das Makrodiol in solchen Mengen verwendet, dass sich im fertigen Polyurethan ein Gewichtsverhältnis von Weichsegment zu Hartsegment von etwa 4 : 1 bis 1 : 4 einstellt.

2. Verfahren nach Anspruch 1, dadurch gekennzeichnet, dass man als monomere, niedermolekulare Diole Alkylendiole mit 2 bis 4 Kohlenstoffatomen verwendet.

3. Verfahren nach Anspruch 1 bis 2, dadurch gekennzeichnet, dass man die Ausgangsstoffe in einer Konzentration von etwa 15 - 30 Gew.-% verwendet.

4. Verfahren nach den Ansprüchen 1 bis 3, dadurch gekennzeichnet, dass man als aromatisches Diisocyanat 4,4'-Diphenylmethandiisocyanat verwendet.

5. Verfahren nach den Ansprüchen 1 bis 4, dadurch gekennzeichnet, dass man als Katalysator Salze einer aromatischen Monocarbonsäure verwendet.

6. Verfahren nach Anspruch 5, dadurch gekennzeichnet, dass man als Katalysator Salze der Benzoesäure verwendet.

7. Verfahren nach Anspruch 5, dadurch gekennzeichnet, dass man als Katalysator Salze der Pyridin-2-carbonsäure verwendet.

8. Verfahren nach Anspruch 5, dadurch gekennzeichnet, dass man als Katalysator Salze der Thiophen-2-carbonsäure verwendet.

9. Verfahren nach den Ansprüchen 1 bis 8, dadurch gekennzeichnet, dass man als inertes Lösungsmittel γ-Butyrolacton verwendet.

10. Verfahren nach den Ansprüchen 1 bis 8, dadurch gekennzeichnet, dass man als inertes Lösungsmittel Dimethylacetamid verwendet.

11. Verfahren nach den Ansprüchen 1 bis 10, dadurch gekennzeichnet, dass man als Markodiol Polyalkylenglykole verwendet.

12. Verfahren nach Anspruch 11, dadurch gekennzeichnet, dass man als Polyalkylenglykol Polyäthylenglykol verwendet.

13. Verfahren nach Anspruch 11, dadurch gekennzeichnet, dass man als Polyalkylenglykol Polytetramethylenglykol verwendet.

14. Verfahren nach den Ansprüchen 2 bis 13, dadurch gekennzeichnet, dass man als niedermolekulares, monomeres Diol Äthylenglykol verwendet.


**Claims for the Contracting States:**
BE, CH, FR, GB, IT, LI, LU, NL, SE

1. A process for the production of linearly segmented polyurethanes by simultaneously reacting macrodiols, low molecular weight diols as chain extenders and aromatic diisocyanates in a solvent in the presence of catalysts, characterized in that macrodiols, monomeric low molecular weight diols and aromatic diisocyanates are reacted at around 70 to 120°C in an inert solvent in the presence of catalytic quantities of a magnesium or calcium salt of an aromatic carboxylic acid, the concentration of the starting materials amounting to between about 10 and 40% by weight, based on the mixture of starting materials and solvent, and the macrodiol being used in such quantities that the ratio by weight of soft segment to hard segment in the final polyurethane is between about 4 : 1 and 1 : 4.

2. A process as claimed in claim 1, characterized in that alkylene diols containing from 2 to 4 carbon atoms are used as the monomeric low molecular weight diols.

3. A process as claimed in claims 1 and 2, characterized in that the starting materials are used in a concentration of from about 15 to 30% by weight.

4. A process as claimed in claims 1 to 3, characterized in that 4,4'-diphenyl methane diisocyanate is used as the aromatic diisocyanate.

5. A process as claimed in claims 1 to 4, characterized in that salts of an aromatic monocarboxylic acid are used as the catalyst.

6. A process as claimed in claim 5, characterized in that salts of benzoic acid are used as the catalyst.

7. A process as claimed in claim 5, characterized in that salts of pyridine-2-carboxylic acid are used as the catalyst.

8. A process as claimed in claim 5, characterized in that salts of thiophene-2-carboxylic acid are used as the catalyst.

9. A process as claimed in claims 1 to 8, characterized in that $\gamma$-butyrolactone is used as the inert solvent.

10. A process as claimed in claims 1 to 8, characterized in that dimethyl acetamide is used as the inert solvent.

11. A process as claimed in claims 11 to 10, characterized in that polyalkylene glycols are used as the macrodiol.

12. A process as claimed in claim 11, characterized in that polyethylene glycol is used as the polyalkylene glycol.

13. A process as claimed in claim 11, characterized in that polytetramethylene glycol is used as the polyalkylene glycol.

14. A process as claimed in claims 2 to 13, characterized in that ethylene glycol is used as the low molecular weight monomeric diol.

15. Polyurethanes obtained by the process claimed in claims 1 to 14, characterized by a molecular inconsistency U of the polyurethanes of $\overline{M}_w : \overline{M}_n = 2\text{-}7$

16. Polyurethanes as claimed in claim 15, characterized by a molecular inconsistency U of 2-5.

17. Polyurethanes as claimed in claim 15 or 16, characterized by an average molecular weight $\overline{M}_w$ of from 60,000 to 250,000.

18. Polyurethanes as claimed in claim 17, characterized by an average molecular weight $\overline{M}_w$ of from 100,000 to 150,000.

19. The use of the polyurethanes claimed in claims 15 to 18 for the production of membranes.

20. The use claimed in claim 19 for the production of membranes for sterile filtration.

21. The use claimed in claim 19 for the production of membranes for the oxygenation of blood.

**Claims for the Contracting State: AT**

1. A process for the production of linearly segmented polyurethanes by simultaneously reacting macrodiols, low molecular weight diols as chain extenders and aromatic diisocyanates in a solvent in the presence of catalysts, characterized in that macrodiols, monomeric low molecular weight diols and aromatic diisocyanates are reacted at around 70 to 120°C in an inert solvent in the presence of catalytic quantities of a magnesium or calcium salt of an aromatic carboxylic acid, the concentration of the starting materials amounting to between about 10 and 40% by weight, based on the mixture of starting materials and solvent, and the macrodiol being used in such quantities that the ratio by weight of soft segment to hard segment in the final polyurethane is between about 4 : 1 and 1 : 4.

2. A process as claimed in claim 1, characterized in that alkylene diols containing from 2 to 4 carbon atoms are used as the monomeric low molecular weight diols.

3. A process as claimed in claims 1 and 2, characterized in that the starting materials are used in a concentration of from about 15 to 30% by weight.

8. A process as claimed in claim 5, characterized in that salts of thiophene-2-carboxylic acid are used as the catalyst.

9. A process as claimed in claims 1 to 8, characterized in that $\gamma$-butyrolactone is used as the inert solvent.

10. A process as claimed in claims 1 to 8, characterized in that dimethyl acetamide is used as the inert solvent.

11. A process as claimed in claims 11 to 10, characterized in that polyalkylene glycols are used as the macrodiol.

12. A process as claimed in claim 11, characterized in that polyethylene glycol is used as the polyalkylene glycol.

13. A process as claimed in claim 11, characterized in that polytetramethylene glycol is used as the polyalkylene glycol.

14. A process as claimed in claims 2 to 13, characterized in that ethylene glycol is used as the low molecular weight monomeric diol.

**Revendications pour les Etats contractants:**
BE, CH, FR, GB, IT, LI, LU, NL, SE

1. Procédé pour la préparation de polyuréthanes à segments linéaires par réaction simultanée de macrodiols de diols à bas poids moléculaire comme allongeurs de chaîne et de diisocyanates aromatiques, dans un solvant, en présence de catalyseurs, caractérisé par le fait qu'en présence de quantités catalytiques d'un sel de magnésium ou de calcium d'un acide carboxylique aromatique on fait réagir des macrodiols, des diols monomères à bas poids moléculaire et des diisocyanates aromatiques à une température d'environ 70 à 120°C dans un solvant inerte, la concentration des matières de départ étant d'environ 10 à 40% en poids par rapport au mélange de matières de départ et de solvant, et qu'on utilise le macrodiol en quantité telle que dans le polyuréthane fini il s'établit un rapport pondéral du segment mou au segment dur d'environ 4 : 1 à 1 : 4.

2. Procédé selon la revendication 1, caractérisé par le fait qu'on utilise comme diols monomères à bas poids moléculaire, des alkylènediols ayant 2 à 4 atomes de carbone.

3. Procédé selon les revendications 1 et 2, caractérisé par le fait qu'on utilise les matières de départ en une concentration d'environ 15 à 30% en poids.

4. Procédé selon les revendications 1 à 3, caractérisé par le fait qu'on utilise comme diisocyanate aromatique, le 4,4'-diphénylméthanediisocyanate.

5. Procédé selon les revendications 1 à 4, caractérisé par le fait qu'on utilise comme catalyseur, des sels d'un acide monocarboxylique aromatique.

6. Procédé selon la revendication 5, caractérisé par le fait qu'on utilise comme catalyseur, des sels de l'acide benzoïque.

7. Procédé selon la revendication 5, caractérisé par le fait qu'on utilise comme catalyseur, des sels de l'acide pyridine-2-carboxylique.

8. Procédé selon la revendication 5, caractérisé par le fait qu'on utilise comme catalyseur, des sels de l'acide thiophène-2-carboxylique.

9. Procédé selon les revendications 1 à 8, caractérisé par le fait qu'on utilise comme solvant inerte, la γ-butyrolactone.

10. Procédé selon les revendications 1 à 8, caractérisé par le fait qu'on utilise comme solvant inerte, le diméthylacétamide.

11. Procédé selon les revendications 1 à 10, caractérisé par le fait qu'on utilise comme macrodiol, les polyalkylèneglycols.

12. Procédé selon la revendication 11, caractérisé par le fait qu'on utilise comme polyalkylèneglycol, le polyéthylèneglycol.

13. Procédé selon la revendication 11, caractérisé par le fait qu'on utilise comme polyalkylèneglycol, le polytétraméthylèneglycol.

14. Procédé selon les revendications 2 à 13, caractérisé par le fait qu'on utilise comme diol monomère à bas poids moléculaire, l'éthylèneglycol.

15. Polyuréthanes obtenus d'après l'un des procédés selon les revendications 1 à 14, caractérisés par une hétérogénéité moléculaire des polyuréthanes de $U = \overline{M}_w : \overline{M}_n = 2 - 7$.

16. Polyuréthanes selon la revendication 15, caractérisé par une hétérogénéité molaire de $U = 2 - 5$.

17. Polyuréthanes selon les revendications 15 ou 16, caractérisé par un poids moléculaire moyen $\overline{M}_w = 60\,000$ à $250\,000$.

18. Polyuréthanes selon la revendication 17, caractérisé par un poids moléculaire moyen $\overline{M}_w = 100\,000$ à $150\,000$.

19. Utilisation des polyuréthanes selon les revendications 15 à 18, pour la préparation de membranes.

20. Utilisation selon la revendication 19, pour la préparation de membranes pour la stérilfiltration.

21. Utilisation selon la revendication 19, pour la préparation de membranes pour l'oxygénation du sang.

**Revendications pour l'Etat contractant: AT**

1. Procédé pour la préparation de polyuréthanes à segments linéaires par réaction simultanée de macrodiols de diols à bas poids moléculaire comme allongeurs de chaîne et de diisocyanates aromatiques, dans un solvant, en présence de catalyseurs, caractérisé par le fait qu'en présence de quantités catalytiques d'un sel de magnésium ou de calcium d'un acide carboxylique aromatique on fait réagir des macrodiols, des diols monomères à bas poids moléculaire et des diisocyanates aromatiques à une température d'environ 70 à 120°C dans un solvant inerte, la concentration des matières de départ étant d'environ 10 à 40% en poids par rapport au mélange de matières de départ et de solvant, et qu'on utilise le macrodiol en quantité telle que dans le polyuréthane fini il s'établit un rapport pondéral du segment mou au segment dur d'environ 4 : 1 à 1 : 4.

2. Procédé selon la revendication 1, caractérisé par le fait qu'on utilise comme diols monomères à bas poids moléculaire, des alkylènediols ayant 2 à 4 atomes de carbone.

3. Procédé selon les revendications 1 et 2, caractérisé par le fait qu'on utilise les matières de départ en une concentration d'environ 15 à 30% en poids.

4. Procédé selon les revendications 1 à 3, caractérisé par le fait qu'on utilise comme diisocyanate aromatique, le 4,4'-diphénylméthanediisocyanate.

5. Procédé selon les revendications 1 à 4, caractérisé par le fait qu'on utilise comme catalyseur, des sels d'un acide monocarboxylique aromatique.

6. Procédé selon la revendication 5, caractérisé par le fait qu'on utilise comme catalyseur, des sels de l'acide benzoïque.

7. Procédé selon la revendication 5, caractérisé par le fait qu'on utilise comme catalyseur, des sels de l'acide pyridine-2-carboxylique.

8. Procédé selon la revendication 5, caractérisé par le fait qu'on utilise comme catalyseur, des sels de l'acide thiophène-2-carboxylique.

9. Procédé selon les revendications 1 à 8, caractérisé par le fait qu'on utilise comme solvant inerte, la γ-butyrolactone.

10. Procédé selon les revendications 1 à 8, caractérisé par le fait qu'on utilise comme solvant inerte, le diméthylacétamide.

11. Procédé selon les revendications 1 à 10, caractérisé par le fait qu'on utilise comme macrodiol, les polyalkylèneglycols.

12. Procédé selon la revendication 11, caractérisé par le fait qu'on utilise comme polyalkylèneglycol, le polyéthylèneglycol.

13. Procédé selon la revendication 11, caractérisé par le fait qu'on utilise comme polyalkylèneglycol, le polytétraméthylèneglycol.

14. Procédé selon les revendications 2 à 13, caractérisé par le fait qu'on utilise comme diol monomère à bas poids moléculaire, l'éthylèneglycol.